(19) Europäisches Patentamt
European Patent Office
Office européen des brevets

(11) Publication number: **0 513 604 A1**

(12) **EUROPEAN PATENT APPLICATION**

(21) Application number: **92107446.4**

(22) Date of filing: **30.04.92**

(51) Int. Cl.[5]: **G01N 33/28**, C10L 1/00, C10M 171/00

(30) Priority: **03.05.91 US 695409**

(43) Date of publication of application:
**19.11.92 Bulletin 92/47**

(84) Designated Contracting States:
**AT BE DE ES FR GB IT NL**

(71) Applicant: **NALCO CHEMICAL COMPANY**
**One Nalco Center**
**Naperville Illinois 60563-1198(US)**

(72) Inventor: **Brinkman, Kerry, C.**
**8907 Tavistock Drive**
**Houston, Texas 77031(US)**
Inventor: **Reese, Dwaine D.**
**2206 Country Creek Way**
**Richmond, Texas 77469(US)**
Inventor: **Bode, Heinreich E.**
**2400 Old South Drive &NUM; 292**
**Richmond, Texas 77469(US)**

(74) Representative: **Ruschke, Hans Edvard, Dipl.-Ing. et al**
**Pienzenauerstrasse 2**
**W-8000 München 80(DE)**

(54) **Labeling of hydrocarbons.**

(57) A population of thousands of hydrocarbons, liquids or gases can be traced from a point of sampling, and back to the originating source by employing combinations of labeling compounds in a base 2 or higher coding system to give $M^n$-1possible codes where n is the number of labelers and M is a variable such as concentration, enabling any specific hydrocarbon thus sampled to have its provenance verified in terms of origin or quality; monitoring gasolines at motorist service station sites is a notable example of practice, but other hydrocarbon liquids and gases may be traced.

EP 0 513 604 A1

## Field of the Invention

This invention involves identification of hydrocarbon fluids (both gases and liquids) using a base 2 or higher encoding system in order to establish or trace provenance. The term provenance is used in the sense of origin, form or quality or the hydrocarbon to be traced.

## Background of the Invention

Environmental agencies are addressing "non-attainment" areas, meaning compacted metropolitan areas, the largest cities, where motor vehicle emissions have not yet attained established environmental standards. The requirement is or will be that motor fuels for consumers, within a given metropolitan radius, will be modified to reduce hydrocarbon emissions and achieve cleaner burning by revising the hydrocarbon distribution and by incorporation of certain oxygenates. The addition is to improve octane rating, to produce a smoother burning fuel and to improve combustion. The addition of oxygenates such as methyl tertiarybutyl ether may be somewhere in the range of one-half to twenty percent by volume depending on the degree of non-attainment. In any event, these enhanced fuels will be more expensive. The possibility for substitution of a less costly non-attainment fuel arises, so close checks become important.

Responsibility lies with the refiner of which there are many sources. The refiner may employ what is believed to be a superior aromatic or a superior oxygenate, a superior mixture or an optimum amount to meet standards. The refiner wants to be assured that any condemned non-compliance fuel is the responsibility of another; in short, the refiner wants to be assured that what is pumped is what that refiner sold, that if there is failure in compliance or other quality it is a matter of counterfeiting, tampering or cheating by another. The present invention addresses the need to effectively establish provenance not only for a large number of producers and refiners but for a large number of different hydrocarbons by each source.

There are numerous other examples as to which provenance becomes important, from the standpoint of "not ours", if nothing else: the source or non-source of an oil spill, oil well leakage or unauthorized dumping; whether or not someone is surreptitiously diluting high octane with a lower octane; whether low grade motor oils are being substituted for a high grade label, or whether low quality lube fractions are employed to degrade a high priced lube of high quality, and sold at the higher price; whether low grade natural gas is being mixed with high grade methane or a natural gas otherwise degraded in terms of BTU's; whether waste dry cleaning fluid or some other cheap octane degrader is being dumped into a storage tank containing high octane gasoline, and so on.

Considering then the order of hydrocarbons (well-head crude production and transportation of crudes, gasolines, diesel fuels, higher alkanes or alkenes for organic synthetics, lube frictions and natural gas to list partially) and then to take into account the numerous producers and refiners, the various octanes or other grades, and modifications such as non-attainment grades, it can be visualized that tagging or labelling for provenance is of enormous magnitude.

Assume five producers, five hydrocarbons, three grades or quality ratings for each, and just three "non-attainment" modifications among the five. The possibilities are

$5 \times 5 \times 3 + 5 \times 3$ equal to 90.

The tag or label disclosure according to U. S. Patent No. 4,141,692 is consequently not the answer. While that disclosure does propose labeling to identify or distinguish sources, spills and co-mingling by employing tracer compounds susceptible to chromatographic-electron capture detection (CR/ECD), the concept there falls short of a binary system of detection featured under the present invention. Further, the compounds chosen for sensitivity according to Patent No. 4,141,692 are a disparity of chloro-compounds which are not only corrosive upon combustion, but are environmentally unacceptable.

## Summary of the Invention

If a different detectable label or tag is to be assigned to each hydrocarbon liquid, the result is that if there are 100 different liquids then one must rely on 100 different labels. This of course falls short of the plain fact that the number of hydrocarbon liquids will be much larger in the instance of supervising for or monitoring ten different producers or refiners.

In accordance with the present invention the labeling compounds, to establish identity, are arranged in a base 2 (binary) or higher matrix system $M^n$-1 where n is the number of labeling compounds and M is an

independent variable such as concentration. M relates to the number of possible values the independent variable can assume for a particular system. For example, when the variable is concentration and the system is base 2 (binary), then M has 2 possible values: 0 and 1. When the number is 0, the label is absent. When the number is 1, the label is present at a concentration high enough to be detected by the chosen analytical method.

| Number of Unique Labeling Compounds | Number of Codes (base 2) |
|---|---|
| n | $2^n$-1 |

Therefore, simply by employing 10 compounds, the possible unique combinations of the ten that can be used to identify liquids is 1024-1 = 1023 which indeed is close to reality, because the invention first of all addresses the reality of the number of domestic producers and refiners who are or will be concerned. Under the binary system, there is room for the practice to be extended to many, many non-domestic producers and refiners as well.

Thus, to extend, if n = 15, the possibilities are 32768-1 = 32767. It therefore becomes possible to meet the needs of many producers and refiners throughout the world, or many different levels in the entire distribution system.

In addition, with precise application of a labeling package and precise analysis of the labeled liquid, dilutions of the liquid can be detected. All packages would be dosed so that, upon analysis, the labels would produce a known theoretical response by the detector. Any response less than the expected value would indicate the liquid was diluted with a different liquid.

In practice, the producer or distributor is given a package of labeling compounds to be mixed with the hydrocarbon products on a species specific basis. Assuming, for simplicity, four labeling compounds (A, B, C, D) and that the producer/refiner markets a crude oil (code 1), a natural gas (code 2), four lube grades (codes 3-7), four octanes (codes 8-12), two diesel fuels (13, 14) and finally a non-attainment blend (15), a binary-based system under the present invention would be as follows:

TABLE 1

| Hydrocarbon Code | Labeling Substances D (8's) | C (4's) | B (2's) | A (1's) |
|---|---|---|---|---|
| 1 | 0 | 0 | 0 | 1 |
| 2 | 0 | 0 | 1 | 0 |
| 3 | 0 | 0 | 1 | 1 |
| 4 | 0 | 1 | 0 | 0 |
| 5 | 0 | 1 | 0 | 1 |
| 6 | 0 | 1 | 1 | 0 |
| 7 | 0 | 1 | 1 | 1 |
| 8 | 1 | 0 | 0 | 0 |
| 9 | 1 | 0 | 0 | 1 |
| 10 | 1 | 0 | 1 | 0 |
| 11 | 1 | 0 | 1 | 1 |
| 12 | 1 | 1 | 0 | 0 |
| 13 | 1 | 1 | 0 | 1 |
| 14 | 1 | 1 | 1 | 0 |
| 15 | 1 | 1 | 1 | 1 |

Reserves

| 16 ' ' ' (127) | G (64ths) | F (32nds) | E (16ths) |
|---|---|---|---|

Having adopted the labeling system, and the hydrocarbon/label equivalents being known, it becomes then a matter of monitoring in the market place. Samples are collected in the field at the point of use or concern. The samples are returned to an authorized or certified analytical laboratory where the master codes are under security. The hydrocarbon and label are separated (e.g. chromatography). The separated label is subjected to any analysis by which an electrical signal analog is generated, registered (displayed) and compared to the codes in the master file of the laboratory to establish (or disestablish) provenation which may be source alone or source plus quality as will be described in more detail.

Homologs

Preferably the labeling compounds are homologs. Homologs are defined here as a series of chemical compounds which all contain the same molecular core and differ only by a substituent or substituents which are attached to the core molecule. The substituent, for example, an alkyl radical may be bound directly to the core through a carbon-carbon bond by a process of alkylation, acylation or nucleophilic substitution or be bound through a heteroatom such as oxygen, nitrogen, sulfur or phosphorus. Functional groups such as acetals and ketals, amides, amines, azos, carbamates, carbonates, carboxylic esters, disulfides, ethers, hydrazines, hydrazones, imides, imines, phosphonates, phosphates, sulfides, sulfonamides, sulfonic acid esters, ureas and others are appropriate for attaching substituents to a core molecule. The substituents can be anything that will alter the physical or chemical properties of the core molecule in a unique way so that

all of the homologs will have a slightly different property that will allow them to be separated from each other by a separation technique such as chromatography. The substituents should not significantly alter those properties of the core segment that allow it to be observed at low concentrations by some analytical technique. For example, if a label is to be observed by a fluorescence detector attached to an HPLC (high pressure liquid chromatograph) separation system, the substituents on the core molecule should not cause significant variations in the emission characteristics of the series. A series of homologs is preferred over a series of unique and unrelated labeling molecules for two reasons.

First, from a manufacturing standpoint, it is far easier to produce a series of related compounds than a series of unrelated ones. For homologs, the core substance can be purchased or manufactured, then derivatized in a single step. A complete series of labeling molecules can be made simply by attaching a series of alkyl radicals of varying sizes to the core molecule through an ester, amide, ether, amine or other easily formed linkage. On the other hand, each unique and unrelated labeling molecule would have to be manufactured by a costly multi-step synthesis.

Second, homologs are preferred because the physical and chemical properties of each label can be made to be very similar. This is important because it greatly simplifies the analytical procedure when the labeling molecules come out of the separation system sequentially and in close proximity to each other as opposed to at widely varying times which may occur with unrelated labeling molecules. Having the labeling molecules detected sequentially and in close proximity aids in the visualization of the binary code and more readily lends itself to full automation of the detection and data processing aspects of the system.

Homologs with similar physical and chemical properties are also more easily separated as a whole unit from the liquid they label. Under many circumstances, the liquid will contain unrelated substances that interfere with detection and the labels must be separated from the liquid before passing through the detector. It would be very difficult to guarantee that tracer molecules with widely varying properties could all be separated from the liquid by a single technique. Finally, the criteria for a labeling molecule to be compatible with the liquid to which it is added, and compatible with the application for which the liquid is intended can be very severe. It is very difficult to identify a series of unrelated molecules that meet all of the separability, detectability and compatibility requirements for a given application. Since homologs have similar physical and chemical properties, these considerations are greatly simplified.

However, while homologs are preferred for the aforementioned reasons, it is understood that there are cases where the use of unrelated molecules in a labeling system provides adequate results, and thus the digital coding system is not restricted to homologs.

Prefarably the labeling compounds (solutes) are homologs. Seven homologs that provide $2^7$-1 unique labels (for example) are easier to manufacture than 1023 tracers which are not homologs, to say nothing of detection disparity. Further, both a chromatograph, or other separator, and an analog detector would be easier to assemble and operate for detection of a series of homologs compared to labels of widely variant chemistry.

The advantage of homologs can be best explained in terms of gas chromatography as the preferred technique for separation. In gas chromatography the term "distribution coefficient" is often employed to characterize separation of a compound from the carrier traveling through the chromatograph column. Two different compounds will elute from the column at different times; they are distributed differently between the carrier gas (mobile phase) and the polymeric stationary phase when traveling through the column and this difference in distribution is what accounts for their eluting or leaving the column at different times. The distribution difference is effected by (1) their volatility; (2) polarity; (3) the temperature of the column; (4) the manner or mode of packing the column (e.g. with polydimethylsiloxane as a separation medium) so the different compounds will have varying distribution coefficients; (5) the diameter of the column, and (6) the flow rate. It is therefore easier to operate a gas chromatographic system for related compounds than unrelated compounds because the distribution coefficients will have a predictable range.

## Brief Description of the Drawing

Fig. 1 is a diagrammatic view of system application under the present invention;
Fig. 2 is a schematic view of the preferred analytical procedure;
Figs. 3A through 3D are diagrammatic illustrations of the results of analyses;
Fig. 4 is another depiction of an analysis;
Figs. 5A and 5B show how the binary system of census may be enlarged; and
Fig. 6 is a diagram of fluorometric analysis.

## Preferred Embodiments of the Invention

Under the present invention, the preference is for a base 2 encoding scheme for fluids (liquids and gases) which employs a family of homologs as the labeling compounds. Whenever it is desired to determine the code number of an unknown sample of labeled fluid, the homologs are preferably separated from the fluid and from each other by a separator device then detected by an analytical instrument or instruments. The detected signal is preferably recorded as peaks along a time axis that can be integrated and correlated to concentration (M).

More preferably, the invention is a binary encoding scheme where a value of 0 is recorded when the concentration of each homolog is too low to be detected and a value of 1 is recorded when the concentration is high enough to be detected. The separation device is more preferably based upon chromatography.

Most preferably, the separation device is a gas chromatograph and the analytical instrument is an electron capture detector.

The labeling compounds or indicators are most preferably homolog ester derivatives of 3,5-dinitrobenzoic acid. This allows an extended number "n" to be more easily achieved from a manufacturing standpoint compared to labels which are not homologs. These esters are non-toxic, environmentally inert, and are non-corrosive as to engine performance and catalytic converters. They exhibit detection sensitivity at least as low as 1 ppm in hydrocarbon fluids and gases (solvents). Moreover, they exhibit similarly shaped ECD peak profiles so that the binary sequence is sharp and unambiguous.

The esters in the homologous series may be: ethyl, propyl, butyl, pentyl, hexyl, isobutyl and isopentyl subscribing to $n = 7$. But n can obviously be higher simply by expanding the number of esters. For purposes of this disclosure, the esters may be taken as ester one (E1) and so on through ester seven, E7. They will elute from a gas chromatograph column sequentially at times $t_1$ through $t_7$, in a file so to speak, and will alter the base line voltage of an electron capture detector (ECD) as the ultimate signature or fingerprint. Optionally, the ECD can be arranged in series or parallel with a mass spectrograph to identify unambiguously the labels as they elute from the chromatography column. The binary code is readily visualized as time-spaced ordinals, that is, ECD peaks spaced along a time axis as will be explained.

Referring to Fig. 1, the authentic source of the hydrocarbon to be traced is identified by reference character 10. The source may be a well-head producer (crude oil is the product to be traced) or it may be the distillation product of a refinery or a distributor.

At the source 10 a sample 5 is taken and sent to the certified laboratory 12 where the binary code of the sample is stored in bank 1. The source material for commercial distribution is transported along a path 14 to an ultimate destination 16. This path may be a pipeline, a high seas tanker, an overland semi-trailer or even an underground leak. To determine provenance and integrity at the destination, a sample S' is taken and returned to the laboratory, bank 2, where the analysis is to be made and compared to the original code stored at the laboratory. The results of comparison are reported to the owner or operator of the source.

Gas chromatography and electron capture detection are the preferred analytical procedures for reasons mentioned. These are well-known analytical procedures described in numerous texts and publications: Handbook of Derivatives for Chromatography (Karl Blau and Graham King); Modern Practice of Gas Chromatography (Robert L. Grob).

In Fig. 2, the analytical procedure is diagrammed and described in very general terms. The sample S', Fig. 1, is introduced by a syringe 20 to the injector 22 of the chromatograph column 22C. The injector is heated as by a coil 24. An inert carrier gas 26 is forced into the injector to mix with the labeled hydrocarbon to be identified.

The hydrocarbon liquid (crude, gasoline, etc.) and labeling substances are vaporized in the injector and are carried into the column by the carrier gas. The less polar and more volatile hydrocarbon molecules interact less with the polymeric stationary phase in the column and pass through to the detector quickly. The more polar and less volatile labeling compounds move through the column more slowly and separate from the hydrocarbon. If two or more molecular labels from the hydrocarbon are present and have different alkyl radical substituents, they will interact with the stationary phase (medium) slightly differently and will have slightly different retention times ($R_t$) within the column and subsequently will elute from the column sequentially. The ester homologs of 3,5-dinitrobenzoic acid are good electrophores and hence easily and unambiguously alter the ECD baseline voltage.

The eluted label solutes are therefore passed out of the chromatograph column to the detector, preferably an ECD detector 30 having current leads 31 to a recorder R.

Referring once again to Table 1, the hydrocarbon codes may be illustrated as the presence or absence of the members of a family of the ester homologs. To present an example of $n = 4$ four esters) it is sufficient for purposes of disclosure, to assign the esters as follows:

TABLE 2

| Ester of 3,5 di-nitrobenzoic Acid | | Binary Code Assignment | Elution Time (arbitrary) |
|---|---|---|---|
| 1. | ethyl (E1) | 0001 | $t_1$ |
| 2. | propyl (E2) | 0010 | $t_2$ |
| 3. | E1 + E2 | 0011 | $t_1,t_2$ |
| 4. | butyl (E3) | 0100 | $t_3$ |
| 5. | E1 + E3 | 0101 | $t_1,t_3$ |
| 6. | E2 + E3 | 0110 | $t_2,t_3$ |
| 7. | E1 + E2 + E3 | 0111 | $t_1,t_2,t_3$ |
| 8. | pentyl (E4) | 1000 | $t_4$ |
| 9. | E4 + E1 | 1001 | $t_1,t_4$ |
| 10. | E4 + E2 | 1010 | $t_2,t_4$ |
| 11. | E4 + E2 + E1 | 1011 | $t_1,t_2,t_4$ |
| 12. | E4 + E3 | 1100 | $t_3,t_4$ |
| 13. | E4 + E3 + E1 | 1101 | $t_1,t_3,t_4$ |
| 14. | E4 + E3 + E2 | 1110 | $t_2,t_3,t_4$ |
| 15 | E4 + E3 + E2 + E1 | 1111 | $t_1,t_2,t_3,t_4$ |

Consequently, a choice of only four esters (ethyl, propyl, butyl, pentyl) allows the fifteen identifications listed in TABLE 2.

Typical analyses are depicted in Figs. 3A, 3B, 3C and 3D. The "unknowns", the samples returned for verification, are S1, S2, S3 and S4. Symbol R designates the recorder. The vertical axis is voltage v; the horizontal axis is time t. The dashed line emitting from the electron capture detector (ECD) symbolizes the time-spaced voltage analogs of the labels transmitted to the recorder, screen display or sheet print-out.

Consider first unknown S1. Its voltage analogs (v), determined, registered and displayed or recorded, are spaced as ordinals along the time axis, horizontal ordinals $t_1$, $t_2$, $t_3$. The sample S1 is therefore binary number 0111 (ethyl + propyl + butyl) which translates to the Arabic numeral 7. The source may be AA company.

Consider sample S2, an ordinal only at $t_2$. The binary equivalent is 0010. The decimal equivalent is 2. The identification or fingerprint for the hydrocarbon is propyl. The source may be BB company.

Suppose that AA company has only been given the label 1000, pentyl ester, assigned to 92 octane. Suppose someone has access to a supply of 87 octane produced by BB company, (propyl label) and uses it to dilute 92 octane of AA company, all labeled as noted.

AA Company (92 octane) - label 1000

BB Company (87 octane) - label 0010

When the retail sale is sampled on behalf of AA and the code elutes as 1010 which is not assigned AA, that company is immediately informed of the dilution. This is shown in Fig. 3D sample S4.

The illustrations just considered assume the hydrocarbons to be identified are injected at the source with solute labels at the same molar concentration or concentrations that will produce a predetermined response by the detector. As a consequence, the areas under the voltage peaks at $t_1$, $t_2$, etc. will be equal and may be assigned the value of x; see Fig. 4. It may be mentioned in this connection the preferred labeling compounds are effective at 1 part per million. This feature of equal concentration (x) itself permits counterfeiting to be recognized because the ECD peaks can be integrated by the analyzer for the area under the peak, the peaks being the voltage analogs at ordinals $t_1$, $t_2$, etc.

An example, and one to be purposely chosen in administering the system, is to assign a binary digit common to the same (or different) octanes of two companies AA and BB:

AA Octane 92 - 1001

BB Octane 92 - 1010

If these octanes from the two different sources should be mixed in equal portions without authority, for whatever reason, sample S5, Fig. 4, the voltage analog peak at the $t_4$ position will be twice the area of the others. This can be visually discerned on the recorder screen or print-out, leaving no doubt that at the site where the sample was taken someone substituted or mixed AA and BB octane 92. This information is of benefit to both companies.

The binary code system greatly enlarges the ability quickly and accurately to take a census of an industrial hydrocarbon population where the population is in the thousands.

Other Encoding Schemes

While the most preferred embodiment of the invention employs the labeling molecules in a digital (base 2) code, it is understood that the same molecules could be employed in such a way to create a code in base 3, base 4, and so on. In the base 2 system, all of the homologs are initially employed at concentrations that produce an equal response (equal area under the graphed peak) upon detection. However, the concentrations of the homologs could be selected so as to produce many discrete levels of response that are multiples of the level or value of the response obtained in the base 2 system.

This scheme can be visualized from Fig. 5. According to Fig. 5A which shows an example of the base 2 system with $2^n$-1possible codes, the labeling components are injected at the same molar concentration, x. All peak areas are the same and the binary read-out is readily visualized as 10111. For this binary number five identifiers would be used (n = 5).

In Fig. 5B, five identifiers are again employed, but in a base 4 scheme, the identifiers are present in concentrations of 0, x, 2x and 3x to give four possible values for each identifier. The code, while still using five identifiers becomes 13203, and from this it can be seen how a system of n identifiers can be transformed to $M^n$-1 possible codes where M stands for the base number and the number of concentration levels possible including the zero level. Thus, in binary base 2, M can be zero (0) concentration (not present at all), or present (1). Base M in terms of concentration (not present, or present) is therefore binary base 2, i.e., M = 2. If there are merely three levels as shown, the possibilities are $4^5$-1 = 1023. If we revert to the seven esters (and there are many more), then $M^n$-1 ($4^7$-1) becomes 16,383 possible codes. Table 1 (seven identifiers) is thus expanded from the low hundreds to many thousands.

While coding schemes above base 2 offer more possible codes, complications arise if the labeled liquid is diluted or mixed with another labeled liquid. A code of 02002 would read as 01001 if the labeled liquid were diluted by 50 percent and the identification would be erroneous. Thus, the binary scheme is preferred because it provides unambiguous results.

Other Analytical Procedures

While gas chromatography with an electron capture detector (ECD) is the preferred basis for a coding system that employs a series of homologs because it is very amenable to automation, it is understood that there are many other analytical systems that would serve equally well as a basis for a multi-component labeling system for liquids. One that is comparable to the preferred system is high pressure liquid chromatography (HPLC) with a fluorescence detector. Whereas the preferred system employs a family of derivatives of an ECD sensitive core molecule, fluorescent molecules can also be derivatized to give families of homologs. These homologs would elute from the HPLC column sequentially and at the predictable times $t_1$ through $t_n$ whereupon they would be detected at the predetermined emission wavelength by the fluorescence detector. Optionally, the intensity of the emissions could be recorded and compared to a theoretical value to determine if a labeled sample had been diluted. Based upon the presence or absence of emissions at $t_1$ through $t_n$, the labeling code would be discovered and the sample identity could be ascertained.

Other types of chromatography are also amenable to the separation of the labeling compounds such that they assume unique values that can be correlated to the compounds. Thin layer chromatography, for example, measures the unique distance that a compound elutes along a thin layer of separation media when a solvent is allowed to flow along the layer. The distance can be measured as a function of time or as the ratio of the distance traveled by the compound to the distance traveled by the solvent front. Each unique distance, $d_1$ through $d_n$, can be assigned a place value in a binary number with n digits in a manner analogous to the elution times $t_1$ through $t_n$. Detection of the presence of a label at d is usually accomplished by the visual observation of a color that is different from the background separation media. The color may become apparent when exposed to light of the appropriate wavelength or require the use of a developing agent that reacts with the labeling compound.

In the case of fluorescence, it is understood that two independent variables such as the elution time (t) for a compound to pass through an HPLC and its fluorescent wavelength could be employed to create a code in base 2 and higher. For example, several (n) different homologous families of fluorescent dyes could be employed in a single application if several (n) fixed wavelength detectors were connected in series to monitor the output of an HPLC separation system or if a single detector could rapidly scan and record the entire emission spectra. Each fixed detector would be tuned to register only the wavelength unique to one of the families of homologs and would be blind to the emissions from the other families at different wavelengths. Each homologous family would be analyzed to determine the time (t) after injection at which

each homolog of each family passes through and is registered by the appropriate detector. The resulting coding system can be described by the formula $M^n$-1where M is the number of possible selections within each family including the possible absence of any homolog from that family in the fluid (i.e., if there are 9 homologs then M = 10) and n is the number of homologous families with different emission wavelengths. For example, if four dyes with widely separated emission bands were each derivatized with 9 alkyl radicals of various lengths, then thirty-six compounds would be made. The homologs and their corresponding elution times would represent "integers" while the wavelengths, families and corresponding detectors would represent the powers or place values. Thus, with a maximum of four compounds in any one package (one from each family) then $10^4$-1 codes are available (0001 through 9999). This example is a base 10 system and benefits from a large number of possible codes with only a few labeling substances added to any one application. It suffers from the larger number of labeling substances that must be made available from which to select and the additional complexity of the detection and decoding system.

From the preceding examples it should be apparent that almost any modern analytical technique could be adapted to detect the presence or absence of small concentrations of a family of tracer substances that might be in a labeled liquid. Upon the determination of the presence or absence of each of the members of the tracer family, the digital code can be discovered and the identity or source of the liquid can be ascertained.

Example 1

If an oil company wanted a way to ensure that all the gasoline sold at its franchised stations came only from its refinery and the octane grades were never mixed, then a digital coding system based upon the presence or absence of specific labeling substances could be employed.

For example, ten homologs of 3,5-dinitrobenzoic acid could be made by esterifying the acid with methanol, ethanol, propanol, butanol and so on through decanol. All ten homologs would be passed through a gas chromatograph equipped with an electron capture detector. The time (t) that it took from injection until each homolog was registered by the detector as a change in the baseline voltage would be determined. The methyl ester, eluting at time $t_1$, would be assigned the place value of 0000000001 (1). The ethyl ester at time $t_2$ would have the place value of 0000000010 (2). The propyl ester ($t_3$) would have the value of 0000000100 (4). The butyl ester ($t_4$) would be 0000001000 (8). The pentyl ester ($t_5$) would be 0000010000 (16). The hexyl ester ($t_6$) would be 0000100000 (32). The heptyl ester ($t_7$) would be 0001000000 (64). The octyl ester ($t_8$) would be 0010000000 (128). The nonyl ester ($t_9$) would be 0100000000 (256) and the decyl ester ($t_{10}$) would have the place value of 1000000000 (512).

A unique number between 1 and 1023 ($2^{10}$-1) would be assigned to each octane grade level of gasoline manufactured by each major producer. Suppose a code of 1000000011 (515) was assigned to a particular company's regular grade, 1000011000 (536) to its mid-grade and 1011000000 (704) to its premium grade of gasoline. Then, a solution containing the methyl (1), ethyl (2) and decyl (512) esters of 3,5-dipitrobenzoic acid would be added to the regular grade either at the refinery or at the tank truck-loading rack at an equimolar concentration of, say, 1 to 5 ppm of each ester in the gasoline. The mid-grade would receive the butyl (8), pentyl (16) and decyl (512) esters while the premium grade of gasoline would receive the heptyl (64), octyl (128) and decyl (512) esters, also at equimolar concentrations in the 1 to 5 ppm range.

If customer complaints targeted a particular station, then small samples of each of the grades of gasoline would be collected at the pump and analyzed with a gas chromatograph and an electron capture detector. Suppose upon analysis of the regular grade, peaks were observed at $t_1$, $t_2$, and $t_{10}$ and the integration ratio relative to the theoretical values ware 0.79, 0.78 and 0.79 respectively. Analysis of the mid-grade shows peaks at $t_4$, $t_5$ and $t_{10}$ with integrations of 0.98, 1.00 and 1.01 respectively. Analysis of the premium grade shows peaks at $t_1$, $t_2$, $t_7$, $t_8$ and $t_{10}$ with integrations of 0.24, 0.23, 0.70, 0.69 and 0.94 relative to theoretical. This data would indicate that the appropriate regular gasoline with a code of 1000000011 (515) was being used but that it was diluted approximately 21% with an unlabeled hydrocarbon of unknown source or quality. The mid-grade with a code of 1000011000 (536) is also from the appropriate source and appears to be of good integrity because the concentrations of the labels are within experimental error of the theoretical values. The premium grade with code 1011000011 (707) is adulterated as evidenced by a mis-match of the code. A closer examination of the integrations would suggest that the premium was diluted with approximately 30% of the liquid that was found in the regular tank at that station.

Under such circumstances, samples of the gasoline would be submitted to an independent testing lab for more thorough analysis before disciplinary action was taken.

Example 2

Suppose reformulated gasoline is required by the Government to be sold within the city limits of, say, 21 major metropolitan areas and it cost consumers 10 to 15 cents per gallon more than the gasoline sold outside of these areas. The Government would have a major interest in assuring that the cheaper and more polluting fuel would not find its way into these cities to be sold at the higher prices. The Government could require that labeling substances be added to reformulated gasoline and a different set of labeling substances be added to all non-reformulated gasoline. A digital coding system based upon the presence or absence of derivatives of a compound such as 1-[2-(2-hydroxyethoxy)phenyl]-2-phenyl ethanedione could meet this need as well as provide information on the source of suspect fuels.

For example, ten homologs of 1-[2-(2-hydroxyethoxy)phenyl]-2-phenyl ethanedione could be made by esterifying the hydroxyethoxy appendage with ten different linear and branched carboxylic acids so as to give ten esters with unique and non-overlapping elution times ($t_1$ to $t_{10}$) when analyzed with a gas chromatograph with an electron capture detector. As in Example 1, the homologs and corresponding times $t_1$ to $t_{10}$ would be assigned the base 2 values 0000000001, (1); 0000000010, (2); 0000000100, (4); 0000001000, (8); 0000010000, (16); 0000100000, (32); 0001000000, (64); 0010000000, (128); 0100000000, (256); and 1000000000, (512).

In order to differentiate between reformulated and non-reformulated gasoline, all non-reformulated gasoline could be coded such that it shares a common label that is not used in the re-formulated gasoline. If the label which elutes at $t_1$ were used exclusively and in all non-reformulated gasoline, those codes would all have an odd number (base 10) whereas the reformulated gasoline would all have even numbered codes. The ability to measure accurately the concentrations of the labels would allow any dilutions of re-formulated gasoline with unlabeled hydrocarbons also to be detected.

Suppose upon random sampling of a station in a non-attainment area, a code of 0100101101 (301) was discovered with integration ratios of $t_1$, $t_3$, $t_4$, $t_6$ and $t_9$ equal to 0.36, 0.65, 0.35, 1.00 and 0.64 respectively versus a theoretical 1.0. This would indicate that non-reformulated gasoline with a code of 0000101001 (41) was being illegally added to re-formulated gasoline with a code of 0100100100 (292).

Example 3

It is desirable to label petroleum products that are transported through a pipeline so that a customer is sure they get the material for which they contracted. A digital coding system based upon the presence or absence of the members of a family of homologous fluorescent compounds can be employed. There are numerous fluorescent compounds available commercially that possess functional groups that can be easily derivatized to give a homologous series with the same emission band. For example, Coumarin 339, available from a company such as Kodak, contains a secondary amine group that can be readily alkylated as shown below with alkyl halides, tosylates, triflates and so on. A family of homologs can be created with varying alkyl radical chain lengths that elute sequentially from an HPLC separation device and can be observed at a fixed emission wavelength. The methyl derivative which elutes at time $t_1$ would be assigned the base 2 place value of 0000000001 (1), the ethyl ($t_2$) would have the value of 0000000010 (2), the propyl ($t_3$) would have the value of 0000000100 (4), the butyl ($t_4$) would have the value of 0000001000 (8), the pentyl ($t_5$) would have the value of 0000010000 (16) and so on through decyl ($t_{10}$) which would be assigned the value of 1000000000 (512). The alkylation reaction is:

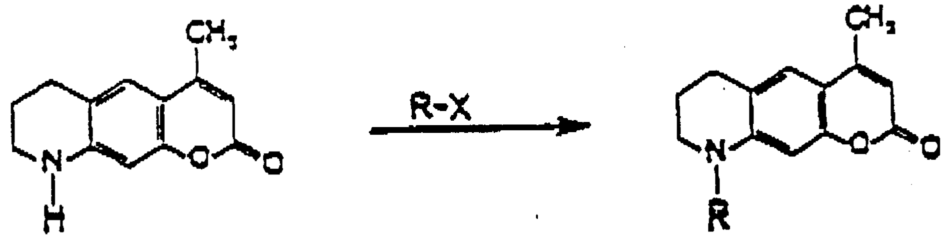


If a company in New England contracted with a refinery on the Gulf Coast for heating oil and wanted to ensure they received the same material they pre-qualified, a solution containing a unique combination of the Coumarin 339 derivatives could be injected into the pipeline as the heating oil was loaded to give a concentration of each derivative of, say, 0.1 ppm. If, for example, the shipment was assigned the base 2 code of 1100000010 (770), then a solution of the decyl (1000000000/512), nonyl (0100000000/256) and ethyl (0000000010/2) derivatives would be used.

When the heating oil reached New England, it would be placed in a storage tank as it came off of the

pipeline. A sample would be analyzed by passing it through an HPLC column to separate the heating oil and separate the homologs of Coumarin 339. The fluorescence detector would register a voltage change each time a derivative passed through it and peaks would be recorded as a function of time. If upon analysis, peaks were recorded at time $t_3$, $t_4$ and $t_7$ but none observed at $t_1$, $t_2$, $t_5$, $t_6$, $t_9$ or $t_{10}$, then a code of 0001001100 (76) would be found and the customer would know they did not have the proper heating oil.

Example 4

Suppose a pipeline operator wanted to label in a unique way each of 500 different petroleum products that were shipped through a section of the pipeline in a year's time, but did not want to add a large number of extraneous substances to each product. A base 10 system employing 9 homologs in each of three different fluorescent dye families would be appropriate. The amine functionality of the fluorescent dyes Carbostyryl 7, Coumarin 151 and Fluoral 555 could be alkylated with methyl through nonyl alkyl groups. Three fluorescent detectors would be connected to an HPLC in series and the first one (units place) would be set at the maxima for the derivatives of Fluorol 555 (about 550 nm), the second detector (tens place) would be set at about 490 nm for the Coumarin 151 homologs and the third detector (hundreds place) wold be set at about 400 nm for the homologs of Carbostyryl 7.

If a code of 301 were selected for a particular product, then the propyl derivative ($t'''_3$) of Carbostyryl 7 and the methyl derivative ($t'_1$) of Fluorol 555 would be injected into the pipeline along with the product to be labeled to give a concentration of each at, say, 1 ppm. The absence of any homolog from the Coumarin 151 family would give the zero value in the tens position (second detector).

Suppose at the other end of the pipeline a sample was analyzed by HPLC and the first detector set at 550 nm registered an emission at a time $t'_9$, the second detector at 490 nm registered an emission at $t''_8$ and the third detector at 400 nm registered an emission at time $t'''_2$. This would be read as a code number of 289 and would result from the nonyl derivative of Fluorol 555, the octyl derivative of Coumarin 151 and the ethyl derivative of Carbostyryl 7. The pipeline operator would then consult the master file to learn more information about this petroleum product.

Analysis for labels employing different fluorescent dye families and homologs thereunder is shown in Fig. 7 where separation of the homologs in any one particular dye family is achieved by the HPLC device.

Hence, while we have illustrated and described the preferred embodiment of the invention in terms of a preferred class of ester homologs, analyzed by gas chromatography and electrophore response, recordable in cartesian coordinates, and where the peaks can be integrated for a value of M (e.g. Fig. 4), it is to be understood there are equivalents for separating and analyzing for a number of labels $l_1...l_n$ in terms of $M^n$-1.

## Claims

1. An identification system for fluids achieved by the addition of a small amount of at least one of n labeling compounds to each fluid at the point of origin for the fluid, being such that the labels are employed in selected permutations conforming to a base M notation where M is the number of possible digital values that a measurable independent variable associated with each of the n labels can assume to produce $M^n$-1 possible unique codes thereby establishing the identity and integrity of different labeled fluids when, subsequently, at the point of destination remote from the point of origin, the fluid is sampled and analyzed by a preselected method that revels the code in base M notation resulting from the measured response for the independent variable of the n labels in the fluid.

2. The system according to claim 1 where the labeling compounds are so selected that the measured response for the variable of the n labels in the fluid is correlated to an electrical response (or lack of response) at discrete retention times $t_1$ through $t_n$, resulting from the length of time that it takes for $label_1$ through $label_n$ to pass through a separation device when a sample of the labeled fluid is passed through the separation device.

3. The system according to claim 2 where the electrical responses at $t_1$ through $t_n$ caused by $label_1$ through labels are chosen to represent specific powers or places of a number with n digits in base M notation.

4. The system according to claim 3 where M is 2 (binary) and the independent variable associated with M is concentration of the labeling compound in binary values 0 and 1 respectively, resulting from the absence or presence of each of the n labels producing a binary number that is specific for the labeled

fluid and imparts identity.

**5.** The system according to claim 3 where M is greater than or equal to 2 resulting from the use of the labeling compounds at variable concentrations producing an electrical response at any of two or more discrete levels.

**6.** The system according to claim 4 where the labels are such that the magnitude and duration of the electrical responses can also be measured and compared to the theoretical values of response that should occur as a result of the concentrations of labels that were added to the fluid at the point of origin and thus determines the integrity of the fluid at the point of sampling.

**7.** The system according to claim 1 where the labeling compounds are so selected that the response for the variable of the n labels in the fluid is correlated to a color at discrete predetermined distances $d_1$ through $d_n$ resulting from the distance that $label_1$ through $label_n$ can travel through a separation medium in a predetermined amount of time with a predetermined elutant, when a sample of the labeled fluid is passed through the separation device.

**8.** The system according to claim 7 where the labeling compounds are so chosen that $d_1$ through $d_n$ associated with $label_1$ through $label_n$ represent specific powers or places of a number with n digits in base M notation.

**9.** The system according to claim 8 where the labeling compounds are so chosen that M is 2 (binary) and the colors at distances $d_1$ through $d_n$ are recorded as not detectable or as detectable in binary notation 0, 1 respectively, resulting from the absence or presence of each of the n labels producing a binary number that is specific for the labeled fluid and imparts identity.

**10.** The system according to claim 7 where the colors result from the labels absorbing or absorbing then emitting light or from a reaction of the labels with a developing agent to produce a color.

**11.** The system according to claim 1 where the n labeling compounds are homologs of one parent compound.

**12.** The system according to claim 1 where M is the number of homologs of a parent compound and n is the number of parent compounds.

**13.** The system according to claim 11 where the homologs are esters.

**14.** The system according to claim 2 where the separation device is a gas chromatograph.

**15.** The system according to claim 2 where the separation device is a liquid chromatograph.

**16.** The system according to claim 7 where the separation device is a thin layer chromatograph.

**17.** The system according to claim 9 where the separation device is a liquid chromatograph.

**18.** The system according to claim 6 where the labels are so chosen that electrical responses are generated as a result of the labels passing through an electron capture detector (ECD).

**19.** The system according to claim 6 where the labels are so chosen that electrical responses are generated as a result of the labels passing through a fluorometer.

**20.** The system according to claim 6 where the labels are so chosen that electrical responses are generated as a result of the labels passing through a mass spectrometer.

**21.** The system according to claim 1 where the fluids to which the labeling compounds are added are selected from the group consisting of gasoline, jet fuel, diesel fuel, heating oil, crude oil, lubrication oil, hydraulic oil, natural gas and liquefied natural gas.

22. The system according to claim 1 where the fluid to which the labeling compound is added is an oxygen-containing blending component of gasoline.

23. The system according to claim 1 where the labeling compounds are premixed with an additive which enhances performance of the fluid before the additive is incorporated in the fluid.

24. The system according to claim 23 where the concentration of the performance-enhancing additive in the fluid is determined by measuring the concentration (magnitude and duration of the electrical response) of the labels in the fluid.

25. A method of imparting identity to fluids which differ as to provenance comprising the following steps:
I. selecting a number, n, of chemically different compounds that are sensitive to and detectable by a preselected analytical instrument and are all separable in a separation device determining the retention times, $t_1$ through $t_n$, or distances, $d_1$ through $d_n$, for each of the compounds in the separation device and assigning a specific place or power of a number with n digits to each of the compound's corresponding retention time or distance, and each compound having one of M concentration levels when added to a fluid;
II. assigning a code number in base M notation to a particular fluid where each digit of the code represents a specific label at one of M possible concentration levels, then blending each of the n labeling compounds into the fluid at the appropriate concentration at a location where the identity and quality is known;
II. collecting a sample of a fluid at another location where its identity and quality are to be determined, and thereafter subjecting the sample to separation followed by analysis by the predetermined method while recording the responses that occur at each of the predetermined retention times or distances, and deciphering the code which is revealed as the absence or presence of a response at each point and the magnitude of that response, the code then being compared to a master list which indicates the expected magnitudes of response to determine the identity and quality of the fluid.

26. The system according to claim 25 where the n labeling compounds are homologs.

27. The system according to claim 26 where the homologs are esters.

28. The system according to claim 26 where the separation device includes a gas or liquid chromatograph.

29. The system according to claim 27 where the separation device includes a gas or liquid chromatograph.

30. The system according to claim 25 where the separation device includes a gas or liquid chromatograph and wherein the retention time or distance responses ar determined by an electron capture detector.

31. The system according to claim 25 where the labels are so selected the responses are electrical responses generated as a result of the labels passing through an electron capture detector.

32. The system according to claim 25 where the labels are so selected the responses are electrical responses generated as a result of the labels passing through a fluorometer.

33. The system according to claim 30 wherein the fluids are selected from the group consisting of gasoline, diesel fuel, crude oil, lubricating oil, heating oil, jet fuel, natural gas, and liquified natural gas.

Fig.1.

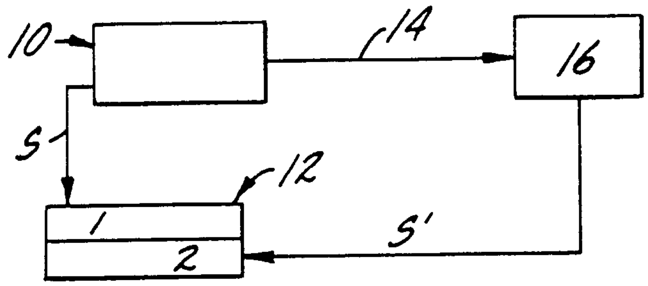


Fig.2.

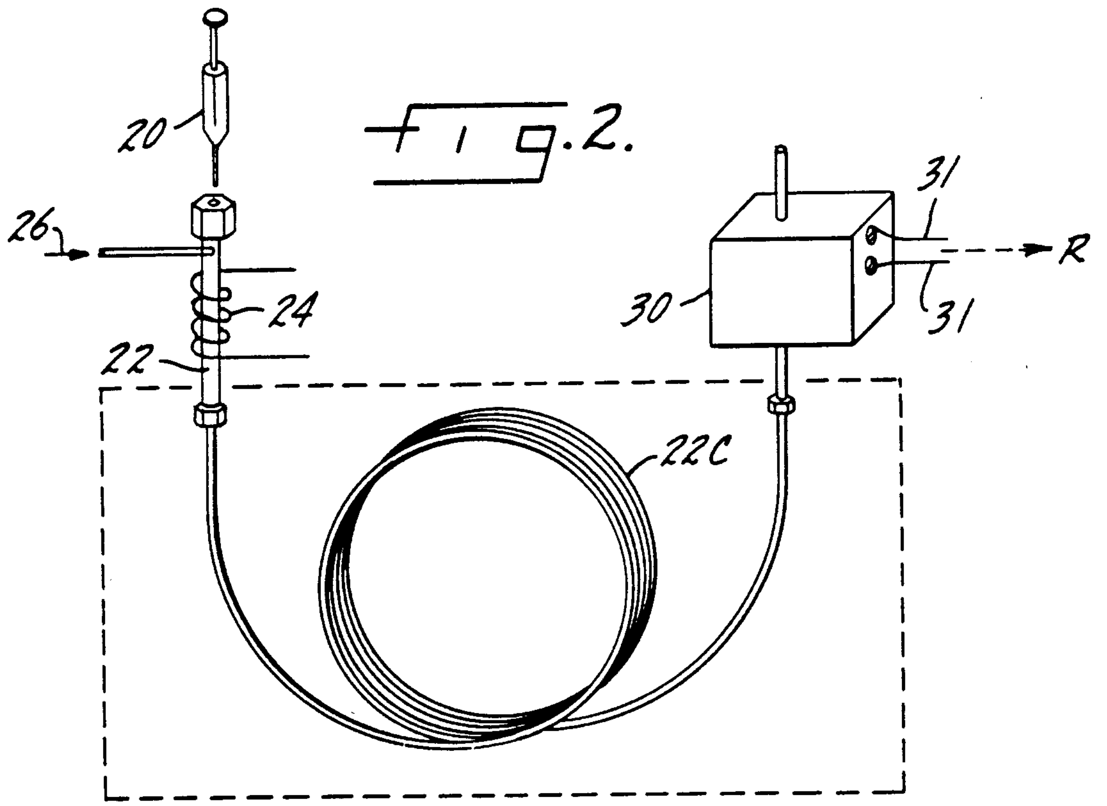


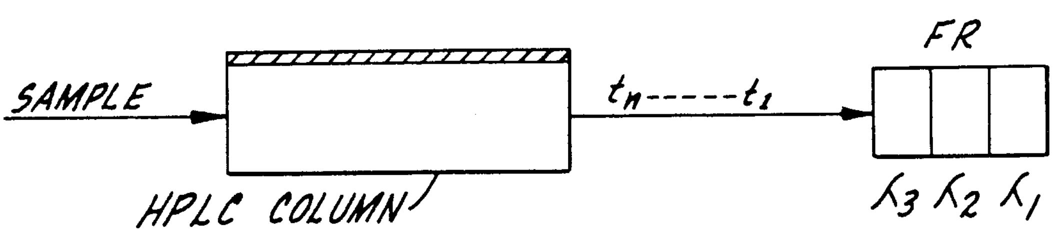


Fig.6.

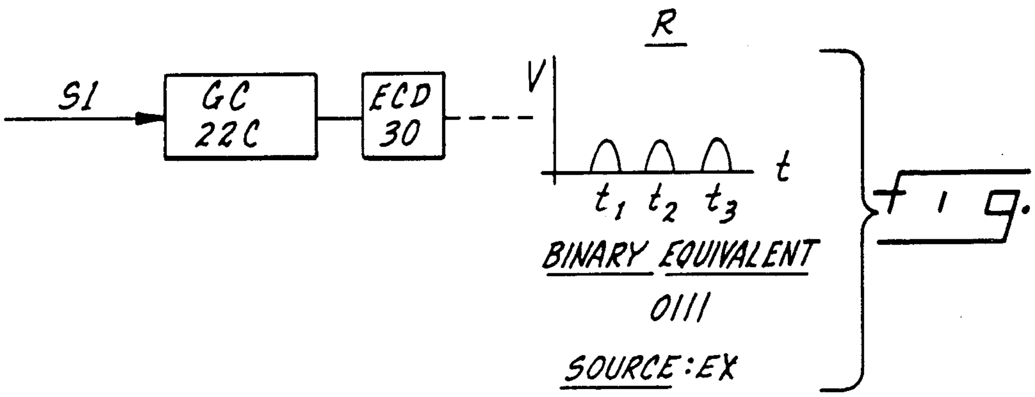


Fig. 3A.

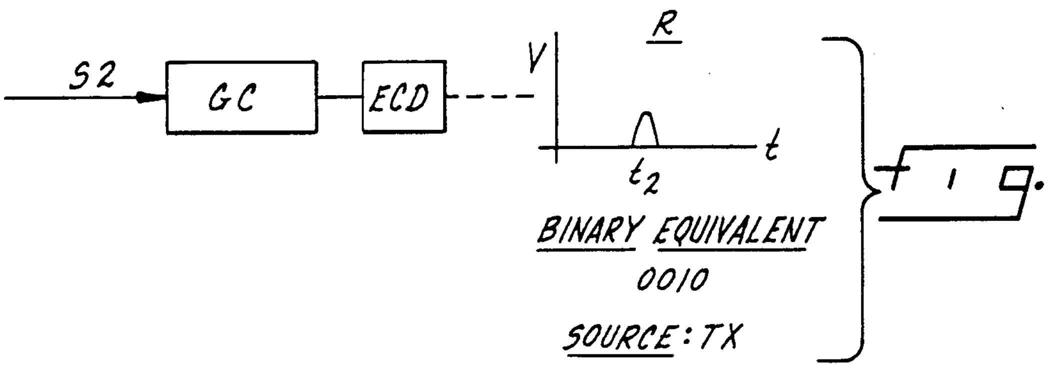


Fig. 3B.

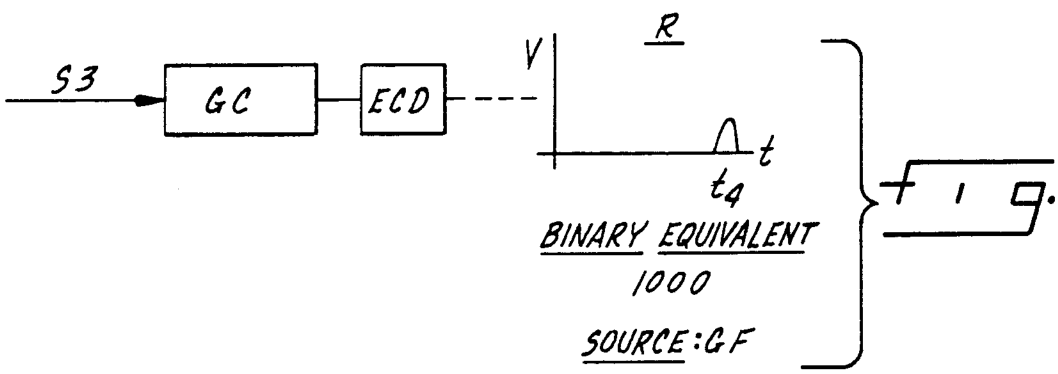


Fig. 3C.

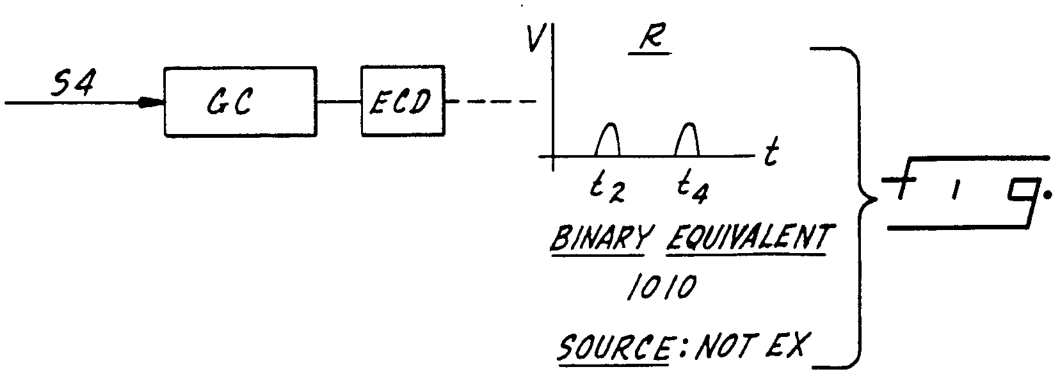


Fig. 3D.

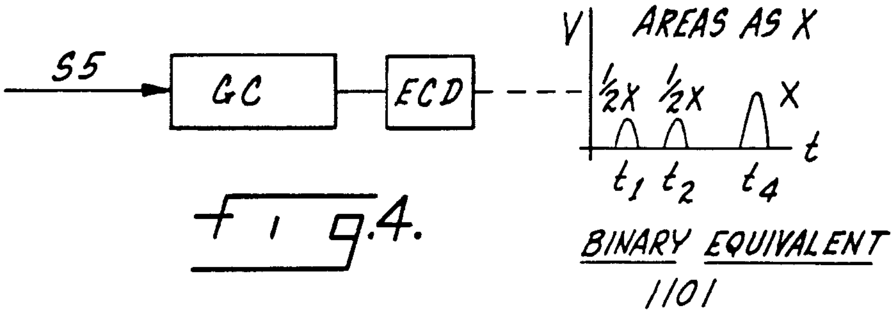


Fig.4.

Fig.5A.

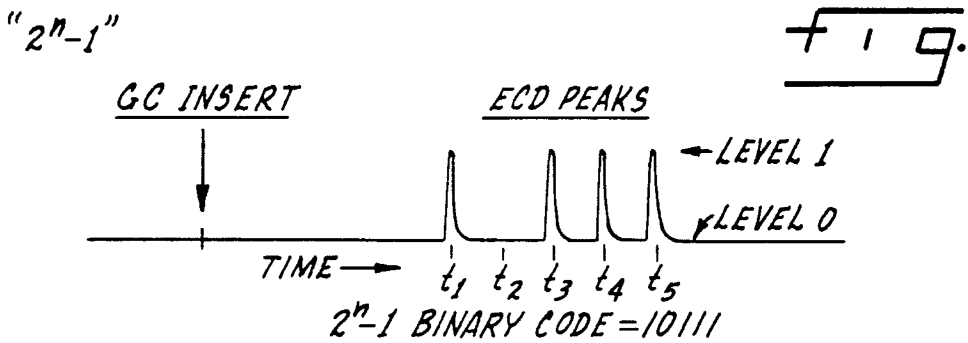


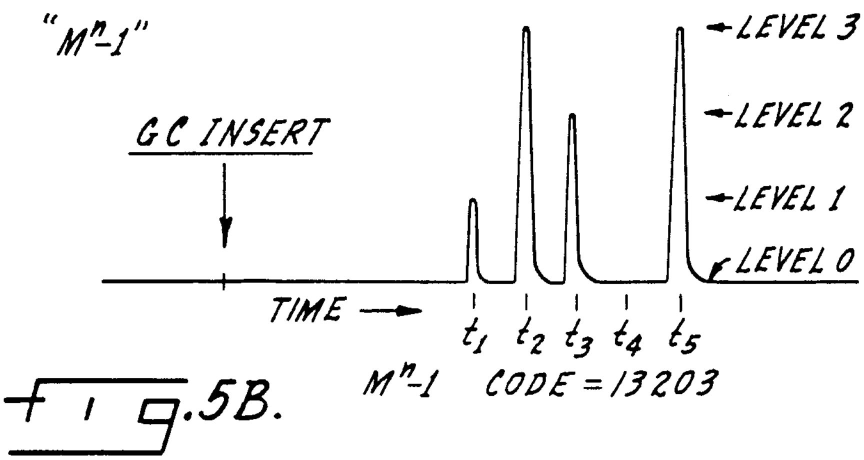


Fig.5B.

European Patent Office

# EUROPEAN SEARCH REPORT

Application Number

EP 92 10 7446
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-3 964 294 (SHAIR ET AL.) | 1-6, 11-15, 18-21, 25-33 | G01N33/28<br>C10L1/00<br>C10M171/00 |
| | * the whole document * | | |
| Y | | 7-10,16, 17,22-24 | |
| | --- | | |
| X | US-A-3 736 500 (BERKOWITZ ET AL.)<br>* the whole document *<br>--- | 1 | |
| Y | JOURNAL OF CHROMATOGRAPHIC SCIENCE.<br>vol. 12, October 1974, NILES, ILLINOIS US<br>pages 564 - 568;<br>GODBILLE ET AL.: 'DESCRIPTION AND PERFORMANCE OF AN 8 CM I.D. COLUMN FOR PREPARATIVE SCALE HIGH PRESSURE LIQUID-SOLID CHROMATOGRAPHY'<br>* figure 5 *<br>--- | 7-9,17 | |
| Y | US-E-16 937 (ISERMAN ET AL.)<br>* the whole document *<br>--- | 22-24 | **TECHNICAL FIELDS SEARCHED (Int. Cl.5 )** |
| Y | US-A-4 918 020 (NOWAK)<br>* the whole document *<br>--- | 7,10,16 | G01N<br>C10L<br>C10M |
| A | GB-A-361 310 (HOWARD FERGUSON)<br>* the whole document *<br>--- | 1-33 | |
| A | US-A-3 154 571 (MIDDLETON)<br>* the whole document *<br>--- | 13,27 | |
| D,A | US-A-4 141 692 (KELLER)<br>* the whole document *<br>--- | 1-33 | |
| A | US-A-4 049 393 (ORELUP)<br>* the whole document *<br>--- | 7-10,16, 21 | |
| | -/-- | | |

The present search report has been drawn up for all claims

| Place of search | Date of completion of the search | Examiner |
|---|---|---|
| THE HAGUE | 27 AUGUST 1992 | DE LA MORINERIE |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................
& : member of the same patent family, corresponding document

European Patent Office

# EUROPEAN SEARCH REPORT

Application Number

EP 92 10 7446
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 147 704 (MORTON THIOKOL, INC.)<br>* the whole document *<br>----- | 7-10,21 | |

TECHNICAL FIELDS SEARCHED (Int. Cl.5 )

The present search report has been drawn up for all claims

| Place of search | Date of completion of the search | Examiner |
|---|---|---|
| THE HAGUE | 27 AUGUST 1992 | DE LA MORINERIE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)